(19) Europäisches Patentamt

European Patent Office

Office européen des brevets

(11) EP 0 688 569 B1

(12) FASCICULE DE BREVET EUROPEEN

(45) Date de publication et mention
de la délivrance du brevet:
**01.09.1999 Bulletin 1999/35**

(51) Int. Cl.⁶: **A61M 1/16**, A61M 1/36,
B01D 71/42, B01D 69/02

(21) Numéro de dépôt: **95420161.2**

(22) Date de dépôt: **16.06.1995**

(54) **Dispositif multifonction pour le traitement du sang**

Mehrzweck-Blutbehandlungsvorrichtung

Multifunction device for blood treatment

(84) Etats contractants désignés:
**AT BE CH DE ES FR GB IT LI NL**

(30) Priorité: **20.06.1994 FR 9407752**

(43) Date de publication de la demande:
**27.12.1995 Bulletin 1995/52**

(73) Titulaire: **HOSPAL INDUSTRIE S.A.**
**F-69883 Meyzieu Cédex (FR)**

(72) Inventeur:
**Pouchoulin, Dominique,**
**Domaine du Grand Pré**
**F-01390 Tramoyes (FR)**

(74) Mandataire: **Tilloy, Anne-Marie**
**IXAS Conseil,**
**15, rue Emile Zola**
**69002 Lyon (FR)**

(56) Documents cités:
**EP-A- 0 100 285**          **EP-A- 0 249 189**
**EP-A- 0 465 380**          **EP-A- 0 592 989**
**DE-A- 2 758 679**

**Description**

L'invention concerne un dispositif multifonction pour le traitement du sang et plus particulièrement pour l'épuration extra-rénale par circulation extra-corporelle du sang.

Différentes méthodes peuvent être utilisées pour éliminer des impuretés contenues dans le sang d'un patient.

L'hémoperfusion : selon cette méthode le sang à traiter s'écoule à travers un agent de sorption, tel du charbon actif, qui retient des impuretés par absorption ou par adsorption. Pour éviter d'endommager le sang, il est courant de recouvrir l'agent de sorption d'une couche hémocompatible perméable aux impuretés, qui empêche le contact direct.

L'hémofiltration et l'hémodialyse : ces deux fonctions sont traditionnellement présentes dans un rein artificiel.

S'agissant de l'hémodialyse, une membrane sépare un courant de sang d'un courant de dialysat ; des impuretés contenues dans le sang à traiter diffusent à travers la membrane vers le dialysat et sont ainsi évacuées.

S'agissant de l'hémofiltration, l'établissement d'une différence de pression de part et d'autre de la membrane force une partie du sang à la traverser. La fraction ayant traversé le filtre, appelée perméat ou ultrafiltrat, contient des molécules ayant une dimension maximale donnée (limite de perméabilité de la membrane) mais aucun des éléments figurés du sang. Le filtrat peut alors être purifié (par exemple par passage sur du charbon actif) avant d'être réintroduit dans le corps du patient. Il est également possible et plus usuel de compenser la perte extra-corporelle liquide et la perte en électrolyte pour le patient au moyen d'une solution de substitution.

Ces diverses méthodes dont l'efficacité n'est plus à démontrer se sont concrétisées par la mise à disposition en milieux hospitaliers de divers types de dispositifs.

La demande de brevet européen EP 0 592 989 divulgue un dispositif multifonction pour le traitement du sang comportant un composant séparateur actif en adsorption présentant une adsorption du TNF-$\alpha$ supérieure à 600ng, le dispositif multifonction ayant un volume de compartiment du dispositif destiné à la circulation de sang de l'ordre de 150ml.

Dans la demande de brevet allemand publiée avant examen sous le n° 2 758 679, il est proposé un dispositif pour le traitement du sang du type " à membrane" dont l'élément actif est constitué d'une couche d'au moins un agent de sorption prise en sandwich par deux membranes semiperméables.

Il est ainsi possible d'intégrer deux des méthodes de traitement du sang, l'hémoperfusion et l'hémofiltration dans un seul appareil. Une fraction du sang est forcée, par application d'un gradient de pression, en tant que filtrat, au travers des trois couches de matériaux : membrane coté sang, couche d'agent de sorption, membrane coté filtrat. Par passage sur la couche intermédiaire, le filtrat est épuré ; des impuretés sont absorbées par ladite couche. La membrane coté filtrat retient également des impuretés.

La demande de brevet britannique n° 2 083 761 propose un dispositif comprenant au moins une unité d'hémodialyse et au moins une unité d'hémoperfusion, montées en série.

L'unité de dialyse peut comporter des fibres creuses ; l'unité d'hémoperfusion est remplie d'une substance absorbante. Les deux unités reliées en série constituent des équipements distincts du dispositif en cause.

Le brevet américain n° 5 194 157 propose une installation de purification du sang comprenant un élément d'hémofiltration monté en série avec un élément d'hémodialyse ainsi qu'un compensateur et un régénérateur.

Ces divers types de dispositifs présentent tous au moins l'inconvénient suivant : dès que l'on souhaite leur faire assumer plus d'une fonction, il est nécessaire de recourir à plusieurs matériaux distincts et, le plus souvent, à plusieurs éléments ou unités physiquement distincts même si ces éléments sont interconnectés.

Par ailleurs, un besoin très net se fait sentir de mettre à la disposition des milieux hospitaliers un dispositif multifonction de conception et d'utilisation relativement simples, pour permettre le traitement des patients dits "aigus", qui ont perdu momentanément, à la suite d'un accident, d'une intervention chirurgicale ou d'un choc septique ou endotoxinique par exemple, tout ou partie de leurs fonctions physiologiques, rénales en particulier.

Ce dispositif devrait permettre notamment d'effectuer les traitements suivants : traitement prophylactique de patients subissant un choc septique, dans la phase précoce (élimination d'eau excédentaire, de TNF-$\alpha$ (facteur de nécrose des cellules tumorales, en anglais, tumor necrosis factor), et d'Interleukine 1-$\beta$); traitement des patients ayant subi une transplantation rénale et soumis à un immunosuppresseur de type OKT3 (élimination d'eau excédentaire et de TNF-$\alpha$) ; traitement des patients ayant subi une chirurgie cardiaque, dans la phase de réchauffage du sang (élimination de TNF-$\infty$ et d'Interleukine 1-$\beta$).

Compte tenu de l'état d'affaiblissement général dans lequel ils se trouvent, les patients "aigus" ne peuvent être soumis aux traitements intensifs que subissent les patients souffrant d'une insuffisance rénale définitive : une séance classique d'hémodialyse ou d'hémofiltration triheodomadaire de quatre heures, par la rapide modification des équilibres liquidiens internes qu'elle provoque, a en effet pour corollaire une sollicitation intense du système cardio-vasculaire que des patients sortant du bloc opératoire ne pourraient, en règle générale, pas supporter.

Pour épurer le sang de ces patients et leur faire éliminer une partie de l'eau qui s'accumule dans leurs tissus , on recourt donc à des traitements peu intenses mais continus, qui sont à la fois bien tolérés par l'organisme car sans à-coups, et supportables par des personnes qui sont hors d'état de se déplacer.

La présente invention a donc pour objet un dispositif multifonction pour l'épuration extra-rénale par circulation extra-corporelle du sang, comportant un composant séparateur semi-perméable constitué par une membrane plane ou un faisceau de fibres creuses, caractérisé en ce que :

a) le composant séparateur est actif en adsorption et présente au moins l'une des capacités d'adsorption ci-après

a1 : adsorption du TNF-$\alpha$ supérieure ou égale à environ 600 ng
a2 : adsorption du facteur D du complément supérieure ou égale à environ 30 mg
a3 : adsorption de l'Interleukine 1-$\beta$ supérieure ou égale à environ 300 ng

b) il présente une clairance de l'urée supérieure ou égale à environ 20 ml/min pour des débits de sang, de liquide de dialyse et d'ultrafiltration égaux respectivement à environ 100 ml/min, 33 ml/min et 0 ml/min et en ce que,
c) il présente un coefficient d'ultrafiltration en présence de sang supérieur à environ 5 ml/(h.mmHg).
d) le volume du compartiment du dispositif destiné à la circulation de sang est inférieur ou égal à environ 150 ml.

Par "dispositif multifonction" on entend un dispositif capable d'exercer simultanément au moins une fonction d'absorbeur, une fonction d'hémodialyseur et une fonction d'hémofiltre.
Par "adsorption" et "adsorber", on entend une diffusion-adsorption, où l'interaction entre les protéines et les chaînes polymériques du matériau du composant séparateur se produisent aussi bien à la surface que dans la masse du matériau.
Les trois capacités d'adsorption définies plus haut correspondent à la quantité totale de TNF-$\propto$, facteur D du complément et/ou d'Interleukine 1-$\beta$ que le composant séparateur a adsorbé lorsqu'il est saturé.
Selon une caractéristique de l'invention, le composant séparateur semi-perméable du dispositif présente au moins deux des capacités d'adsorption définies plus haut et, préférablement, les trois capacités d'adsorption a1, a2 et a3.
Selon une autre caractéristique de l'invention, le composant séparateur présente, pour une efficacité accrue, une ou plusieurs capacité(s) d'adsorption supérieure(s) ou égale(s) aux valeurs limites suivantes :

a1 : adsorption du TNF-$\alpha$ supérieure ou égale à environ 3000 ng
a2 : adsorption du facteur D supérieure ou égale à environ 90 mg, de préférence encore, supérieure ou égale à environ 150 mg
a3 : adsorption de l'interleukine 1-$\beta$ supérieure ou égale à environ 1500 ng

L'invention a également pour objet un dispositif multifonction pour l'épuration extra-rénale par circulation extra-corporelle du sang, du type comportant un faisceau de fibres creuses caractérisé en ce que :

a) les fibres creuses sont composées d'un hydrogel polyélectrolytique, homogène et symétrique dérivant d'un copolymère d'acrylonitrile et de méthallylsulfonate qui renferme environ 3,3 % (molaire) de méthallylsulfonate, et qui est commercialisé par la société HOSPAL sous la marque AN 69 ;

b) la masse totale d'hydrogel contient au moins environ 50g dudit copolymère ;

c) chaque fibre présente un diamètre interne compris entre environ 180 et environ 260 $\mu$m et en ce que

d) le volume du compartiment sang du dispositif est inférieur à environ 150 ml.

Un autre objet de la présente invention est une fibre creuse utile à la réalisation d'un tel dispositif multifonction pour l'épuration extra-rénale par circulation extra-corporelle du sang , cette fibre creuse étant caractérisée en ce que :

a) elle présente un diamètre interne compris entre environ 180 et environ 260 $\mu$m ;

b) elle est composée d'un hydrogel polyélectrolytique, homogène et symétrique ;

c) elle a une perméabilité hydraulique Lp supérieure à environ 5 ml/h/mmHg/m$^2$ ;

d) elle a une résistance membranaire à l'urée inférieure à environ 200 min/cm et en ce que

e) elle a la capacité d'adsorber l'une au moins des substances choisies parmi le TNF-$\alpha$, le facteur D du complément ou l'Interleukine 1-β et sa capacité d'adsorption linéique est, selon le cas, supérieure à environ 125 pg/m pour le TNF-α, supérieure à environ 6 μg/m, de préférence supérieure à environ 30 μg/m, pour le facteur D et supérieure à environ 60 pg/m pour l'Interleukine 1-β.

On va maintenant décrire en détail le dispositif selon l'invention et un type de fibres creuses propre à en constituer le composant séparateur.

Le dispositif selon l'invention comprend deux compartiments séparés par un composant séparateur constitué par une membrane plane semi-perméable ou par un faisceau de fibres creuses semi-perméables. Ce dispositif est multifonction en ce qu'il est apte à exercer simultanément une fonction d'adsorption, définie par une capacité d'adsorption de substances spécifiques, une fonction de dialyse, définie par une clairance de l'urée, et une fonction d'ultrafiltration, définie par un coefficient d'ultrafiltration en présence de sang ou pente au sang.

Le composant séparateur est actif en adsorption en ce sens qu'il présente lui-même au moins l'une des capacités d'adsorption a1 à a3 ci-avant. Il est préférable que ledit composant présente au moins deux des capacités d'adsorption en cause.

Bien entendu, il est avantageux que le composant séparateur présente simultanément les trois capacités d'adsorption a1 à a3.

Pour une meilleure efficacité, le composant séparateur présentera une ou plusieurs capacité(s) d'adsorption supérieure(s) ou égale(s) aux valeurs limites suivantes :

a1 : adsorption du TNF-α supérieure ou égale à environ 3000 ng
a2 : adsorption du facteur D supérieure ou égale à environ 90 mg, de préférence encore, supérieure ou égale à environ 150 mg
a3 : adsorption de l'Interleukine 1-β supérieure ou égale à environ 1500 ng

Le dispositif présente également une clairance de l'urée supérieure ou égale à environ 20 ml/min pour des débits de sang, de liquide de dialyse et d'ultrafiltration égaux respectivement à environ 100 ml/min, 33 ml/min et 0 ml/min.

Cette clairance est spécifiée pour une utilisation du dispositif dans le cadre d'un fonctionnement en hémodialyse continue, en hémodialyse artério-veineuse continue (couramment désignée par CAVHD) ou veino-veineuse continue (couramment désignée par CVVHD), pour le traitement de patients dits "aigus". Elle indique le rendement de l'épuration du dispositif par diffusion des solutés du sang vers le dialysat au travers de la membrane et elle est définie comme le rapport du flux de soluté à la concentration du soluté à l'entrée du dispositif. Elle est déterminée dans le cadre d'un protocole de mesure in vitro, dans lequel le sang est remplacé par une solution d'urée dans le dialysat, la circulation des fluides est conduite à contre-courant et avec une température régulée à 37°C. Sa valeur est fournie pour des conditions de fonctionnement représentatives de celles pratiquées en CAVHD ou CVVHD, soit pour un débit de sang Qs de 100 ml/min, un débit de dialysat Qd de 2 l/h et un débit d'ultrafiltration Qf de 0 ml/min.

Le dispositif présente une pente au sang supérieure à environ 5 ml/(h.mmHg).

La pente au sang ou coefficient d'ultrafiltration est définie comme étant le rapport du débit de filtration Qf à la pression transmembranaire moyenne dans le dispositif diminuée de la pression oncotique. La valeur fournie est déterminée avec le sang circulant à 37°C avec un débit supérieur ou égal à 50 ml/min.

La pente au sang indiquée permet l'extraction d'environ 1 l/h (1 litre/heure) d'hémofiltrat sous 200 mmHg de pression transmembranaire.

Le volume du compartiment du dispositif destiné à la circulation de sang est avantageusement inférieur ou égal à environ 150 ml. Cette valeur est la limite estimée être acceptable compte tenu qu'il est admis que le volume extra-corporel total ,ne devrait pas excéder 250 ml.

Une valeur de l'ordre de 100 ml et, de préférence, de l'ordre de 80 ml est bien entendu encore plus acceptable sur ce plan.

Selon un mode de réalisation du dispositif selon l'invention, le composant séparateur actif en adsorption est conformé à partir d'un seul type de matériau qui est un hydrogel polyélectrolytique, homogène et symétrique, sous forme d'une membrane plane ou d'un faisceau de fibres creuses.

L'hydrogel en cause peut dériver

(1) d'un copolymère de l'acrylonitrile et d'au moins un monomère ionique ou ionisable, renfermant le cas échéant des unités provenant d'au moins un autre monomère à insaturation oléfinique capable d'être copolymérisé avec l'acrylonitrile, ou
(2) d'un copolymère de l'acrylonitrile et d'au moins un monomère ionique ou ionisable et d'au moins un monomère non ionique et non ionisable ou
(3) d'un mélange de copolymères définis sous (1) et/ ou (2) ou

4

(4) d'un mélange d'au moins un copolymère défini sous (1) et/ ou (2) et d'au moins un copolymère de l'acrylonitrile et d'au moins monomère non ionique et non ionisable, les unités de monomère ionique ou ionisable représentant de 1 à 80% (en mole) des unités de monomère de l'un des dits copolymères et représentant de 1 à 50% (en mole) des unités de monomère dans le composé macromoléculaire final.

De tels composés macromoléculaires ainsi que les divers monomères susceptibles d'être retenus comme matières premières de leurs fabrication sont plus amplement décrits dans le brevet américain 4 545 910 redélivré sous le n° Re. 34 239.

Parmi ces composés macromoléculaires, on préfère ceux définis sous (1) ci-avant et, en particulier ceux pour lesquels le comonomère ionique ou ionisable est oléfiniquement insaturé et porteur de groupements anioniques choisis parmi les groupes sulfonate, carboxyle, phosphate, phosphonate et sulfate.

Un monomère particulièrement préféré est le méthallylsulfonate de sodium.

Parmi les monomères à insaturation oléfinique capable d'être copolymérisés avec l'acrylonitrile, on préfère les acrylates d'alkyle et en particulier l'acrylate de méthyle.

L'hydrogel en cause , qui est non réticulé, peut être fabriqué selon le procédé décrit dans le brevet européen n° 0 347 267.

Bien entendu, la nature précise du contre-ion des groupes sulfonates dans l'hydrogel n'est pas essentielle au bon fonctionnement du dispositif selon l'invention : si l'utilisation d'un hydrogel sous forme totalement acide est à proscrire, il est possible qu'une partie soit sous forme acide et/ou , qu'en raison notamment des échanges avec des ions contenus dans le sang tels le potassium et le calcium, ce type de contre-ions soit présent.

On recourt avantageusement à un hydrogel dérivant d'un copolymère d'acrylonitrile et de méthallylsulfonate qui présente une capacité ionique supérieure ou égale à 400 mEq/kg, et de préférence supérieure ou égale à 500 mEq/kg (exprimée par rapport au polymère sec).

Dans le cas particulier où l'on recourt à un hydrogel dérivant d'un copolymère d'acrylonitrile et de méthallylsulfonate qui renferme environ 3,3 % (molaire) de méthallylsulfonate, et qui est commercialisé par la société HOSPAL sous la marque AN 69 (désigné dans ce qui suit par "hydrogel d'AN 69"), il a été mis en évidence que la quantité de protéines adsorbée dépend essentiellement de la masse d'hydrogel présente. Il a pu être déterminé que cette quantité doit être d'au moins environ 50 g (exprimée en polymère sec).

Le composant séparateur actif en adsorption peut être conformé, de manière en soi connue, sous la forme d'une membrane plane ou sous la forme d'un faisceau de fibres creuses.

Les fibres creuses présentent un diamètre intérieur compris entre environ 180 et environ 260 μm.

Dans le cas où celles ci sont constituées d'un hydrogel d'AN 69, leur épaisseur sera comprise entre environ 50 et environ 250 μm.

D'une manière générale, l'épaisseur des fibres creuses constituées à partir d'un hydrogel polyélectrolytique, homogène et symétrique, pourra être comprise entre environ 50 et environ 250 μm. De préférence, elle sera supérieure à 50 μm car, dans ces conditions, on pourra généralement obtenir une augmentation de la capacité d'adsorption en TNF-$\alpha$ et/ou en facteur D du complément et/ou en Interleukine 1-$\beta$.

Comme mentionné plus haut, un autre objet de la présente invention est une fibre creuse utile à la réalisation d'un dispositif multifonction pour l'épuration extra-rénale par circulation extra-corporelle du sang. Cette fibre creuse a les caractéristiques suivantes :

a) elle présente un diamètre interne compris entre environ 180 et environ 260 μm ;

b) elle est composée d'un hydrogel polyélectrolytique, homogène et symétrique ;

c) elle a une perméabilité hydraulique Lp supérieure à environ 5 ml/h/mmHg/m$^2$ ;

d) elle a une résistance membranaire à l'urée inférieure à environ 200 min/cm et en ce que

e) elle a la capacité d'adsorber l'une au moins des substances choisies parmi le TNF-$\alpha$, le facteur D ou l'Interleukine 1-$\beta$ et sa capacité d'adsorption linéique est, selon le cas, supérieure à environ 125 pg/m pour le TNF-$\alpha$, supérieure à environ 6 μg/m, de préférence supérieure à environ 30 μg/m pour le facteur D et supérieure à environ 60 pg/m pour l'Interleukine 1-$\beta$.

La perméabilité hydraulique Lp, l'un des paramètres caractéristiques d'une membrane donnée, est définie comme étant le rapport du débit de filtration obtenu par unité de surface et de pression transmembranaire ; pour les fibres creuses en question, les valeurs de Lp sont indiquées pour un bain de dialyse à 37°C. Il est à noter que lorsque les fibre creuses ont une surface développée supérieure à 1 m$^2$, il y a une correspondance approximative entre la pente au sang minimale mentionnée plus haut et la perméabilité hydraulique minimale au bain de dialyse.

La résistance membranaire à un soluté est définie comme le rapport, en régime permanent, de la différence de concentration de part et d'autre de la membrane au flux de soluté la traversant selon un mode purement diffusif. La mesure directe de la résistance membranaire est rendue difficile par la présence inévitable de résistance de transfert des couches limites parasites du côté sang et du côté liquide de dialyse lors d'une mesure de flux de soluté par diffusion.

Une estimation de la valeur de la résistance membranaire est effectuée à partir des mesures et considérations suivantes :

- la clairance d'un dialyseur est mesurée in vitro, à débit d'ultrafiltration nul ;
- la connaissance des débits et de la surface d'échange permet de calculer la résistance totale de transfert du dispositif à l'aide des lois classiques de l'échangeur purement diffusif.
- cette résistance totale constitue une détermination par excès de la résistance membranaire.

L'hydrogel polyélectrolytique, homogène et symétrique, dont la fibre creuse est composée est identique à celui qui a été décrit plus haut.

S'agissant du diamètre interne, et le cas échéant de l'épaisseur de la fibre creuse, les valeurs précédemment indiquées s'appliquent également.

Les exemples ci- après illustrent l'invention.

**EXEMPLES**

En raison des difficultés inhérentes aux tests in vivo qui ne peuvent être réalisés qu'en milieu hospitalier, et surtout des risques susceptibles d'être encourus par les patients dits "aigus" s'ils étaient soumis à des traitements mettant en oeuvre des dispositifs défectueux, on a procédé à diverses déterminations in vitro.

Pour ce faire, on a choisi une protéine modèle, le cytochrome C, dont le poids moléculaire (12 400) est voisin des cytokines (molécules d'intérêt biologique).

Les exemples 1 à 3 ont pour but de montrer la relation existant entre la capacité d'adsorption de la membrane utilisée et, respectivement, la masse de copolymère contenue dans la membrane, la structure de l'hydrogel dont la membrane est faite et la nature du copolymère.

Les exemples 4 à 6 décrivent respectivement différentes fibres creuses et différents produits.

Pour les exemples 1 à 3, le protocole opératoire suivi pour accéder à la valeur de capacité d'adsorption en équilibre avec la concentration (CO) d'une solution d'une molécule test (cytochrome C) mise en contact avec les hydrogels testés est le suivant :

- les hydrogels testés sont engagés sous forme de membrane plane ou sous forme de fibre creuse ;
- l'échantillon d'hydrogel est introduit dans un récipient contenant un volume Vo de solution de molécule test à une concentration initiale de Co. La solution est agitée.
- comme conséquence au processus d'adsorption, la concentration de la molécule test dans le récipient décroît. Au moyen de dosages et d'ajouts répétés de solution, la concentration de la molécule test dans le récipient est ramenée à plusieurs reprises à la valeur initiale Co, jusqu'à l'obtention d'une concentration quasi stationnaire.
- on détermine alors la capacité d'adsorption de la molécule test à l'isotherme des hydrogels en cause par rapport à la masse de polymère Mp. La quantité adsorbée, déterminée à partir de la masse totale des ajouts successifs (M aj) peut être calculée par la relation suivante, tirée d'un bilan de masse :
   Capacité d'adsorption à l'isotherme de l'hydrogel

$$Cads\ (G)\ iso = [Vo.Co\text{-}Vf.Cf + Maj]/Mp$$

où Vf est le volume final dans le récipient et Cf la concentration finale de molécule test dans le récipient.

<u>Exemple 1</u>

Selon le protocole d'accès à l'isotherme d'adsorption précité, on évalue les capacités d'adsorption des membranes constituées d'hydrogel d'AN 69 qui suivent :

- membrane plane (1a) de 30 $\mu$m d'épaisseur ;

- membrane plane (1b) de 19 $\mu$m d'épaisseur ;

- fibre creuse (1c) de 240 µm de diamètre interne et de 50 µm d'épaisseur de paroi ;

- fibre creuse (1d) de 210 µm de diamètre interne et de 42,5 µm d'épaisseur de paroi.

Préparation des membranes planes (1a) et (1b) :

La composition liquide de départ ou collodion est composée de 20 % en poids d'AN 69 et de 80 % en poids de diméthylformamide (DMF). Les membranes planes (1a) et (1b) sont obtenues par inversion de phase lors d'un coulage à 80°C de ce collodion sur un tambour tournant. Le film dense obtenu est ensuite soumis à un étirage de trois à trois fois et demi dans de l'eau à 90-96° C.

Préparation des fibres creuses (1c) et (1d) :

Elles sont obtenues par un procédé de gélification comprenant le passage d'un collodion composé de 35 % en poids d'AN 69, 52 % en poids de DMF et 13 % en poids de glycérol chauffé à environ 140°C au travers d'une filière. Le filage est réalisé en présence d'un gaz interne de centrage.

Ensuite, les fibres sont soumises à une opération d'étirage de quatre fois à 95 °C.

Résultats des tests d'adsorption

| REF. | 1a | 1b | 1c | 1d |
|---|---|---|---|---|
| Concentration de la solution de cytochrome C (mg/l) | 100 | 100 | 210 | 210 |
| Capacité d'adsorption à l'isotherme Cads (G) iso (mg/g) | 110 | 113 | 595 | 555 |

Pour une structure d'hydrogel donnée, la quantité de protéine adsorbée dépend essentiellement de la masse de copolymère contenue dans la membrane.

Les hydrogels d'AN 69 de structure similaire ont des propriétés d'adsorption identiques si ces propriétés sont rapportées au poids de polymère.

A poids de polymère identique, il faut noter que la membrane plane (1b) développe une surface 1,6 fois plus grande que celle de la membrane plane (1a) (rapport des épaisseurs) et la fibre creuse (1d) une surface 1,2 fois supérieure à celle de la fibre creuse (1c). Le processus d'adsorption a bien lieu dans la masse du matériau et non à la surface apparente de celui-ci.

Exemple 2

Cet exemple illustre l'effet de la structure du gel sur la capacité d'adsorption à l'isotherme de l'hydrogel.

La structure du gel dépend du procédé de fabrication utilisé. Ainsi, les différents procédés mentionnés plus haut ont pour résultat des gels présentant des variantes de structure (teneur en eau, organisation des chaînes de polymère, masse volumique ...).

Matériaux testés

- la membrane plane (1a) décrite ci-avant ;

- la fibre creuse (1c) décrite ci-avant ;

- la fibre creuse d'AN 69 (2e) de 425 µm de diamètre interne et 140 µm d'épaisseur de paroi.

Préparation de la fibre creuse d'AN 69 (2e)

Elle est obtenue par un procédé de coagulation conventionnel impliquant :

- un collodion de 10 % en poids d'AN 69, 5 % en poids de NaCl 9g/l et 85 % de diméthylsulfoxyde (DMSO), qui est filé à température ambiante au travers d'une filière de diamètre égal à 288 μm, la couronne de distribution du collodion présentant les diamètres de 565 μm et 866 μm, sous une vitesse d'appel égale à 9 m/min ;

- un liquide coagulant interne contenant 9 g/l de NaCl ;

- un bac de réception rempli avec le même liquide que le coagulant interne ;

- une absence d'étirage de la fibre ;

- un traitement de la fibre avec un mélange glycérol-eau (teneur en glycérol supérieure à 60 %) en vue de son stockage et de la fabrication ultérieure d'un dispositif.

Résultat des tests d'adsorption

| REF. | 1a | 1c | | 2e |
|---|---|---|---|---|
| | Membrane plane | Fibre creuse | | Fibre creuse |
| Concentration CO de la solution de cytochrome C (mg/l) | 100 | 100 | 210 | 210 |
| Capacité à l'adsorption à l'isotherme Cads (G) iso (mg/g) | 110 | 140 | 595 | 685 |

La comparaison deux à deux des matériaux 1a-1c et 1c-2e issus du même polymère mais de structures assez différentes [ainsi, l'hydrogel (1c) présente une teneur en eau supérieure à celle de l'hydrogel (1a)], montre que la capacité d'adsorption rapportée à la masse de polymère peut être modulée par le choix de la structure du gel.

Outre un effet sur les paramètres de cinétique, la structure de l'hydrogel peut aussi modifier l'accessibilité des sites d'adsorption,

Exemple 3

Dans cet exemple, on a déterminé la capacité d'adsorption du cytochrome C de divers hydrogels dérivant de copolymères de l'acrylonitrile et du méthallylsulfonate de sodium se présentant sous forme de billes, différant par leur teneur en ce dernier monomère et, par suite, présentant différentes capacités ioniques exprimées en mEq/kg de polymère.

Les billes d'hydrogel ont été obtenues par un procédé d'émulsion d'un collodion composé de 10 % en poids de polymère, 6,5 % en poids d'un sérum physiologique (NaCl 9 g/l) et 83,5 % en poids de DMF, cette étape de mise en émulsion étant suivie d'une gélification thermoréversible de l'ambiante vers - 10°C.

Les caractéristiques des hydrogels testés (capacité ionique, teneur en eau) et les résultats des tests d'adsorption d'une solution de 100 mg/l de cytochrome C sont reportés dans le tableau ci-après.

| Capacité ionique (mEq/kg) | 0 | 450 | 580 | 680 | 800 | 1130 |
|---|---|---|---|---|---|---|
| Teneur en eau (% en poids) | 85 | 78 | 78 | 82 | 83 | 90 |
| Cads (G) iso (mg/g) | 28 | 125 | 135 | 165 | 150 | 110 |

La capacité d'adsorption des hydrogels peut être modulée par la nature du polymère en fonction d'un objectif donné d'adsorption vis à vis d'une certaine molécule.

Exemple 4

Cet exemple illustre diverses fibres creuses obtenues par un procédé de coagulation à température ambiante comprenant le passage d'un collodion dont la nature est précisée dans le tableau (N) ci-après, au travers d'une filière de diamètre d1, la couronne de distribution du collodion présentant les diamètres d2 et d3 ; les diamètres d1 à d3 définissant la géométrie de filière sont précisés dans le tableau en cause ; le filage est réalisé en présence d'un liquide coagulant interne dont la composition figure au tableau précité, sous une vitesse d'appel également précisée. Dans certains cas, le filage est réalisé en présence également d'un tube extracteur réalisant une coagulation externe au moyen d'un liquide identique au coagulant interne. La réception des fibres s'opère dans un bac rempli de ce même liquide. Les fibres ne sont pas étirées et, en vue de leur stockage et de la fabrication ultérieure d'un dispositif, elles sont glycérinées avec un mélange glycérol-eau (teneur en glycérol supérieure à 60 %).

Les conditions particulières de fabrication ainsi que les caractéristiques des fibres sont rassemblées dans le tableau (N) ci-après.

TABLEAU (N)

| réf. | 4f | 4g | 4h | 2e |
|---|---|---|---|---|
| Collodion | (1) | (1) | (1) | (2) |
| Coagulant | NaCl 9 g/l | NaCl 9 g/l | Na2HPO4 9 g/l | NaCl 9 g/l |
| Tube extracteur | oui | oui | non | non |
| Géométrie de fillière d1/d2/d3 $\mu$m | 196/240/1020 | 196/240/1020 | 196/240/1020 | 288/565/866 |
| Vitesse d'appel m/min | ≈8,3 | ≈9 | ≈9 | ≈9 |
| diamètre interne des fibres ($\mu$m) | 200 | 240 | 250 | 425 |
| épaisseur de paroi($\mu$m) | 160 | 175 | 180 | 140 |
| masse linéique (mg/ m) | - | 66 | 52 | 44 |
| Lp ml/(h.mmHg.m$^2$)* | 16 | 16 | 23 | 130 |
| Résistance membranaire à l'urée (min/cm)** | <110 | <50 | <65 | <55 |

(1) : AN 69 18% et diméthylformamide 82%

(2) : AN 69 10%, diméthylsulfoxyde 85%, solution à 9g/lde chlorure de sodium 5%

(*) : perméabilité hydraulique au bain de dialyse à 37° C effectuée sur des minifiltres ayant de 50 à 100 fibres.

(**) : résistance membranaire déterminée sur mini-filtres de 50 à 100 fibres à débit sang Qs = 10 ml/min, à débit dialysat Qd = 20 ml/min et à débit nul d'ultrafiltration, le calcul de la résistance de transfert totale constituant une détermination par excés de la résistance membranaire à l'urée.

Exemple 5

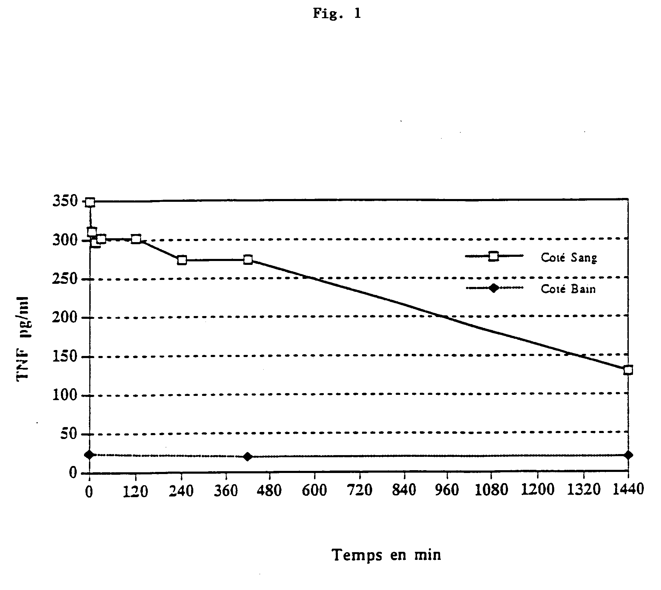

Les propriétés d'adsorption d'un minidialyseur comportant 140 fibres (4g) décrites à l'exemple 4, de 18 cm de longueur ont été évaluées par circulation en circuit fermé de plasma humain chargé en TNF-$\alpha$, la concentration initiale (C0) de cette protéine étant de 350 pg/ml et le volume initial (V0) du plasma étant de 17,5 ml. Aucune circulation de liquide de dialyse à l'extérieur des fibres, ni aucune ultrafiltration ne sont pratiquées. L'évolution de la concentration (C) au cours du temps est montrée par la figure 1 annexée. Une capacité d'adsorption minimale du TNF-$\alpha$ de 2,3 ng par g de polymère peut en être déduite ; pour la fibre (4g), ceci correspond à une capacité d'adsorption linéique supérieure ou égale à 150 pg/m pour le TNF-$\alpha$.

Exemple 6

La capacité d'adsorption pour le facteur D de la fibre (1c), décrite dans les exemples 1 et 2, mesurée in vivo, est supérieure à 4 mg/g d'AN 69, c'est-à-dire supérieure à 56 $\mu$g/m de fibres.

Exemple 7

Sur la base des déterminations qui viennent d'être exposées il est possible de définir des dispositifs dont les fibres creuses sont similaires à celles utilisées pour la réalisation du minidialyseur objet de l'exemple 5, dispositifs dont les caractéristiques sont indiquées dans le tableau ci-après.

| Réf. | Mp (g) | VCS (ml) | Surface (m$^{2)}$) | Pente au sang (1) | Clairance urée (2) |
|------|--------|----------|---------|-------------------|--------------------|
| 7a | 50 | 34 | 0,57 | 9 | ≈25 |
| 7b | 90 | 62 | 1,03 | 16 | ≈30 |
| 7c | 130 | 89 | 1,48 | 23 | ≈32 |
| Mp : masse de polymère | | | | | |
| VCS : volume du compartiment sang | | | | | |

(1) en ml/ (h.mmHg)
(2) en ml/min pour des débits de sang, de liquide de dialyse et d'ultrafiltration égaux respectivement à environ 100 ml/min, 33 ml/min et 0 ml/min.

Exemple 8

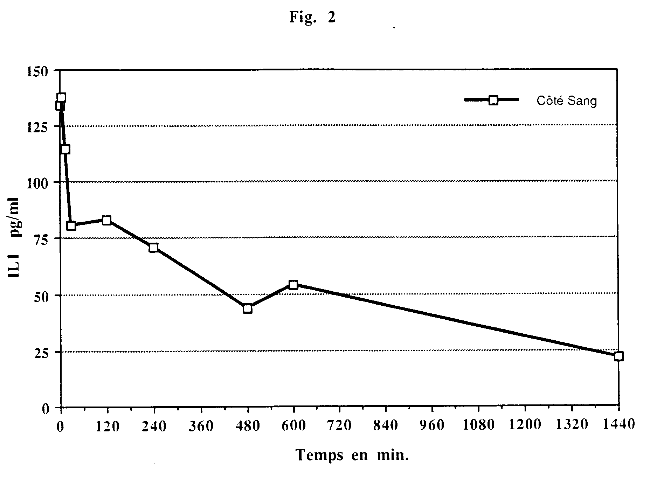

Les propriétés d'adsorption d'un minidialyeur comportant 170 fibres (1c) décrites à l'exemple 1, de 18 cm de longueur ont été évaluées par circulation en circuit fermé de plasma humain chargé en Interleukine 1-β, la concentration initiale (C0) de cette protéine étant de 130 pg/ml et le volume initial (V0) du plasma étant de 20 ml. Aucune circulation de liquide de dialyse à l'extérieur des fibres, ni aucune ultrafiltration ne sont pratiquées. L'évolution de la concentration (C) au cours du temps est montrée par la figure 2 annexée. Une capacité d'adsorption minimale de l'Interleukine 1-β de 4,8 pg par mg de polymère peut en être déduite ; pour la fibre (1c), ceci correspond à une capacité d'adsorption linéique supérieure ou égale à 67 pg/m de fibres pour l'Interleukine 1β.

Exemple 9

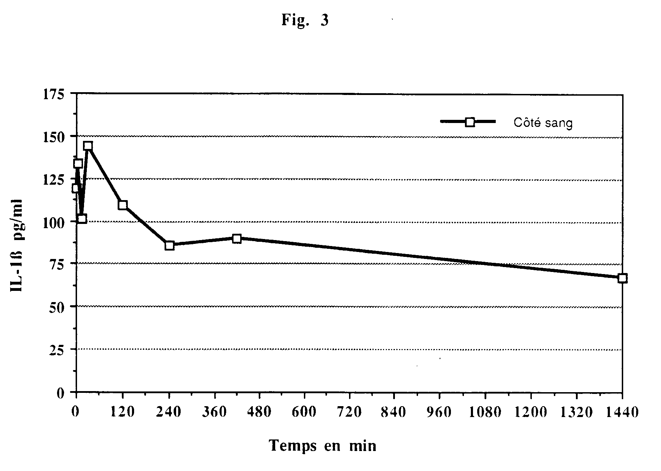

Les propriétés d'adsorption d'un minidialyseur comportant 70 fibres (4g) décrites à l'exemple 4, de 18 cm de longueur ont été évaluées par circulation en circuit fermé de plasma humain chargé en Interleukine 1-β, la concentration initiale (C0) de cette protéine étant de 120 pg/ml et le volume initial (V0) du plasma étant de 20 ml. Aucune circulation de liquide de dialyse à l'extérieur des fibres, ni aucune ultrafiltration ne sont pratiquées. L'évolution de la concentration (C) au cours du temps est montrée par la figure 3 annexée. Une capacité d'adsorption minimale de l'Interleukine 1-β de 1,15 pg/mg de polymère peut en être déduite ; pour la fibre (4g), ceci correspond à une capacité d'adsorption linéique supérieure ou égale à 75 pg/m de fibres pour l'Interleukine 1-β.
Enfin, il convient de noter que :

-  les capacités d'adsorption des exemples 6, 8 et 9 sont mesurées après 24 heures d'essais, sans attendre la saturation des fibres creuses ;
-  les expériences décrites dans les exemples 5, 6, 8 et 9 ont été réalisées à des concentrations en substances à adsorber représentatives des valeurs physiologiques pouvant être atteintes lors de diverses pathologies, telles que celles décrites ci-avant, aux pages 3 et 4 de la présente demande.

**Revendications**

1.  Dispositif multifonction pour l'épuration extra-rénale par circulation extra-corporelle du sang, comportant un composant séparateur semi-perméable constitué par une membrane plane ou un faisceau de fibres creuses, caractérisé en ce que :

    a) le composant séparateur est actif en adsorption et présente au moins l'une des capacités d'adsorption ci-après

       a1 : adsorption du TNF-α supérieure ou égale à environ 600 ng
       a2 : adsorption du facteur D du complément supérieure ou égale à environ 30 mg
       a3 : adsorption de l'Interleukine 1-β supérieure ou égale à environ 300 ng

    b) il présente une clairance de l'urée supérieure ou égale à environ 20 ml/min pour des débits de sang, de liquide de dialyse et d'ultrafiltration égaux respectivement à environ 100 ml/min, 33 ml/min et 0 ml/min

c) il présente un coefficient d'ultrafiltration en présence de sang supérieur à environ 5 ml/(h.mmHg)

d) le volume du compartiment du dispositif destiné à la circulation de sang est inférieur ou égal à environ 150 ml.

2. Dispositif selon la revendication 1, caractérisé en ce que son composant séparateur actif en adsorption présente au moins deux des capacités d'adsorption a1 à a3.

3. Dispositif selon la revendication 2, caractérisé en ce que son composant séparateur actif en adsorption présente toutes les capacités d'adsorption a1 à a3.

4. Dispositif selon l'une quelconque des revendications précédentes, caractérisé en ce que son composant séparateur actif en adsorption présente une capacité d'adsorption du TNF-$\alpha$ supérieure ou égale à environ 3000 ng.

5. Dispositif selon l'une quelconque des revendications précédentes, caractérisé en ce que son composant séparateur actif en adsorption présente une capacité d'adsorption du facteur D supérieure ou égale à environ 90 mg.

6. Dispositif selon l'une quelconque des revendications précédentes, caractérisé en ce que son composant séparateur actif en adsorption présente une capacité d'adsorption du facteur D supérieure ou égale à environ 150 mg.

7. Dispositif selon l'une quelconque des revendications précédentes, caractérisé en ce que son composant séparateur actif en adsorption présente une capacité d'adsorption de l'Interleukine 1-$\beta$ supérieure ou égale à environ 1500 ng.

8. Dispositif selon l'une quelconque des revendications précédentes, caractérisé en ce que son composant séparateur actif en adsorption est conformé à partir d'un seul type de matériau, qui est un hydrogel polyélectrolytique, homogène et symétrique.

9. Dispositif selon la revendication 8, caractérisé en ce que l'hydrogel dérive d'un copolymère de l'acrylonitrile et d'au moins un monomère ionique ou ionisable, renfermant le cas échéant des unités provenant d'au moins un autre monomère à insaturation oléfinique capable d'être copolymérisé avec l'acrylonitrile.

10. Dispositif selon la revendication 8 ou 9, caractérisé en ce que l'hydrogel dérive d'un copolymère d'acrylonitrile et de méthallylsulfonate de sodium.

11. Dispositif selon la revendication 10, caractérisé en ce que la capacité ionique du copolymère est supérieure ou égale à 400 mEq/kg et, de préférence, supérieure ou égale à 500 mEq/kg.

12. Dispositif selon la revendication 10 ou 11, caractérisé en ce que le copolymère renferme environ 3,3 % molaire de méthallylsulfonate de sodium.

13. Dispositif selon la revendication 12, caractérisé en ce que la quantité d'hydrogel qu'il renferme correspond à au moins environ 50 g de copolymère sec.

14. Dispositif selon la revendication 8, caractérisé en ce que les fibres creuses présentent un diamètre intérieur compris entre 180 et 260 $\mu$m, inclus.

15. Dispositif selon la revendication 8, caractérisé en ce que les fibres creuses présentent une épaisseur comprise entre environ 50 et environ 250 $\mu$m.

16. Dispositif selon la revendication 15, caractérisé en ce que les fibres creuses présentent une épaisseur supérieure à 50 $\mu$m.

17. Dispositif selon la revendication 1, lorsque le composant séparateur actif en adsorption est constitué par un faisceau de fibres creuses, caractérisé par des fibres :

a) présentant un diamètre interne compris entre environ 180 et environ 260 µm ;

b) étant composées d'un hydrogel polyélectrolytique, homogène et symétrique ;

c) ayant une perméabilité hydraulique Lp supérieure à environ 5 ml/h/mmHg/m$^2$ ;

d) ayant une résistance membranaire à l'urée inférieure à environ 200 min/cm et

e) ayant la capacité d'adsorber l'une au moins des substances choisies parmi le TNF-$\alpha$, le facteur D du complément ou l'Interleukine 1-$\beta$ et sa capacité d'adsorption linéique est, selon le cas, supérieure à environ 125 pg/m pour le TNF-$\alpha$, supérieure à environ 6 µg/m pour le facteur D et supérieure à environ 60 pg/m pour l'Interleukine 1-$\beta$.

18. Dispositif selon la revendication 17, caractérisé en ce que l'hydrogel dérive d'un copolymère de l'acrylonitrile et d'au moins un monomère ionique ou ionisable, renfermant, le cas échéant, des unités provenant d'au moins un autre monomère à insaturation oléfinique capable d'être copolymérisé avec l'acrylonitrile.

19. Dispositif selon la revendication 17 ou 18, caractérisé en ce que l'hydrogel dérive d'un copolymère d'acrylonitrile et de méthallylsulfonate de sodium.

20. Dispositif selon la revendication 19, caractérisé en ce que la capacité ionique du copolymère est supérieure ou égale à 400 mEq/kg et, de préférence, supérieure ou égale à 500 mEq/kg.

21. Dispositif selon la revendication 19 ou 20, caractérisé en ce que le copolymère renferme environ 3,3 % molaire de méthallylsulfonate de sodium et est commercialisé par la société HOSPAL sous la marque AN 69.

## Claims

1. Multifunction device for extrarenal purification by extracorporeal circulation of blood, including a semipermeable separator component, composed of a plane membrane or a bundle of hollow fibres, characterised in that:

    a) the separator component is active in adsorption and has at least one of the adsorption capacities below:

    a1: adsorption of TNF-$\alpha$ greater than or equal to approximately 600 ng
    a2: adsorption of complement factor D greater than or equal to approximately 30 mg
    a3: adsorption of interleukin 1-$\beta$ greater than or equal to approximately 300 ng

    b) it has a urea clearance greater than or equal to approximately 20 ml/min for rates of blood flow, of dialysis liquid flow and of ultrafiltration equal respectively to approximately 100 ml/min, 33 ml/min and 0 ml/min.
    c) it has an ultrafiltration coefficient in the presence of blood greater than approximately 5 ml/(h.mmHg).
    d) the volume of compartment in the device intended for circulation of blood is less than or equal to approximately 150 ml.

2. Device according to Claim 1, characterised in that its separator component which is active in adsorption has at least two of the adsorption capacities a1 to a3.

3. Device according to Claim 2, characterised in that its separator component which is active in adsorption has all the adsorption capacities a1 to a3.

4. Device according to any one of the preceding claims, characterised in that its separator component which is active in adsorption has a TNF-$\alpha$ adsorption capacity greater than or equal to approximately 3000 ng.

5. Device according to any one of the preceding claims, characterised in that its separator component which is active in adsorption has a factor D adsorption capacity greater than or equal to approximately 90 mg.

6. Device according to any one of the preceding claims, characterised in that its separator component which is active in adsorption has a factor D adsorption capacity greater than or equal to approximately 150 mg.

7. Device according to any one of the preceding claims, characterised in that its separator component which is active in adsorption has a interleukin 1-β adsorption capacity greater than or equal to approximately 1500 ng.

8. Device according to any one of the preceding claims, characterised in that its separator component which is active in adsorption is made from a single type of material which is a homogeneous and symmetrical polyelectrolytic hydrogel.

9. Device according to Claim 8, characterised in that the hydrogel is derived from a copolymer of acrylonitrile and of at least one ionic or ionisable monomer, optionally including units originating from at least one other monomer containing olefinic unsaturation capable of being copolymerised with acrylonitrile.

10. Device according to Claim 8 or 9, characterised in that the hydrogel is derived from a copolymer of acrylonitrile and of sodium methallylsulphonate.

11. Device according to Claim 10, characterised in that the ionic capacity of the copolymer is greater than or equal to 400 mEq/kg, and preferably greater than or equal to 500 mEq/kg.

12. Device according to Claim 10 or 11, characterised in that the copolymer contains approximately 3.3% molar of sodium methallylsulphonate.

13. Device according to Claim 12, characterised in that the quantity of hydrogel which it contains corresponds to at least approximately 50 g of dry copolymer.

14. Device according to Claim 8, characterised in that the hollow fibres have an internal diameter of between 180 and 260 μm, inclusive.

15. Device according to Claim 8, characterised in that the hollow fibres have a thickness of between approximately 50 and approximately 250 μm.

16. Device according to Claim 15, characterised in that the hollow fibres have a thickness of greater than 50 μm.

17. Device according to Claim 1, when the separator component which is active in adsorption is composed of a bundle of hollow fibres, characterised in that the fibres:

    a) have an internal diameter of between approximately 180 and approximately 260 μm;
    b) are composed of a homogeneous and symmetrical polyelectrolytic hydrogel;
    c) have a hydraulic permeability Lp greater than approximately 5 ml/h/mmHg/m$^2$;
    d) have a membrane resistance to urea less than approximately 200 min/cm, and
    e) have the capacity to adsorb at least one of the substances chosen among TNF-α, complement factor D or interleukin 1-β and its linear adsorption capacity is, depending on the case, greater than approximately 125 pg/m for TNF-α, greater than approximately 6 μg/m for factor D and greater than approximately 60 pg/m for interleukin 1-β.

18. Device according to Claim 17, characterised in that the hydrogel is derived from a copolymer of acrylonitrile and of at least one ionic or ionisable monomer, optionally including units originating from at least one other monomer containing olefinic unsaturation capable of being copolymerised with acrylonitrile.

19. Device according to Claim 17 or 18, characterised in that the hydrogel is derived from a copolymer of acrylonitrile and of sodium methallylsulphonate.

20. Device according to Claim 19, characterised in that the ionic capacity of the copolymer is greater than or equal to 400 mEq/kg, and preferably greater than or equal to 500 mEq/kg.

21. Device according to Claim 19 or 20, characterised in that the copolymer contains approximately 3.3% molar of sodium methallylsulphonate, and is marketed by the company HOSPAL under the trademark AN 69.

**Patentansprüche**

1. Multifunktionsvorrichtung zur extrarenalen Reinigung des Bluts durch extrakorporale Umwälzung, enthaltend ein semipermeables Separatorelement, das aus einer ebenen Membran oder aus einem Bündel hohler Fasern besteht, dadurch gekennzeichnet, daß;

    a) das Separatorelement durch Adsorption wirkt und mindestens eine der nachfolgenden Adsorptionskapazitäten aufweist:

        a1: Adsorption des TNF-$\alpha$ größer als oder gleich 600 ng,
        a2: Adsorption des Faktors D des Komplements größer als oder gleich 30 mg,
        a3: Adsorption des Interleukins 1-$\beta$ größer als oder gleich 300 ng,

    b) es für Durchsätze von Blut, Dialyse- und Ultrafiltrationsflüssigkeit, die bzw. gleich ungefähr 100 ml/min, 33 ml/min, 0 ml/min sind, eine Clearance für Harnstoff größer als oder gleich ungefähr 20 ml/min aufweist,
    c) es einen Ultrafiltrationskoeffizienten in Gegenwart von Blut größer als ungefähr 5 ml/(h.mmHg) aufweist,
    d) das Volumen der Kammer der zur Blutumwälzung dienenden Vorrichtung kleiner als oder gleich ungefähr 150 ml ist.

2. Vorrichtung nach Anspruch 1, dadurch gekennzeichnet, daß ihr durch Adsorption wirkendes Separatorelement mindestens zwei der Adsorptionsfähigkeiten a1 bis a3 aufweist.

3. Vorrichtung nach Anspruch 2, dadurch gekennzeichnet, daß ihr durch Adsorption wirkendes Separatorelement alle Adsorptionskapazitäten a1 bis a3 aufweist.

4. Vorrichtung nach einem der vorhergehenden Ansprüche, dadurch gekennzeichnet, daß ihr durch Adsorption wirkendes Separatorelement eine Adsorptionskapazität für TNF-$\alpha$ größer als oder gleich ungefähr 3000 ng aufweist.

5. Vorrichtung nach einem der vorhergehenden Ansprüche, dadurch gekennzeichnet, daß ihr durch Adsorption wirkendes Separatorelement eine Adsorptionskapazität für Faktor D größer als oder gleich ungefähr 90 mg aufweist.

6. Vorrichtung nach einem der vorhergehenden Ansprüche, dadurch gekennzeichnet, daß ihr durch Adsorption wirkendes Separatorelement eine Adsorptionskapazität für Faktor D größer als oder gleich ungefähr 150 mg aufweist.

7. Vorrichtung nach einem der vorhergehenden Ansprüche, dadurch gekennzeichnet, daß ihr durch Adsorption wirkendes Separatorelement eine Adsorptionskapazität für Interleukin 1-$\beta$ größer als oder gleich ungefähr 1500 ng aufweist.

8. Vorrichtung nach einem der vorhergehenden Ansprüche, dadurch gekennzeichnet, daß ihr durch Adsorption wirkendes Separatorelement aus einer einzigen Art Material ausgebildet ist, das ein homogenes und symmetrisches polyelektrolytisches Hydrogel ist.

9. Vorrichtung nach Anspruch 8, dadurch gekennzeichnet, daß das Hydrogel sich von einem Copolymer von Acrylnitril und mindestens einem ionischen oder ionisierbaren Monomer ableitet, das gegebenenfalls konstitutionelle Einheiten enthält, die von mindestens einem weiteren olefinisch ungesättigten Monomer stammen, das mit Acrylnitril copolymerisiert werden kann.

10. Vorrichtung nach Anspruch 8 oder 9, dadurch gekennzeichnet, daß das Hydrogel sich von einem Copolymer von Acrylnitril und Natrium-methallylsulfonat ableitet.

11. Vorrichtung nach Anspruch 10, dadurch gekennzeichnet, daß der Ionengehalt des Copolymers größer als oder gleich 400 mEq/kg, vorzugsweise größer als oder gleich 500 mEq/kg ist.

12. Vorrichtung nach Anspruch 10 oder 11, dadurch gekennzeichnet, daß das Copolymer ungefähr 3,3 Mol-% Natriummethallylsulfonat enthält.

13. Vorrichtung nach Anspruch 12, dadurch gekennzeichnet, daß die Menge Hydrogel, die sie enthält, mindestens 50 g trockenem Copolymer entspricht.

**14.** Vorrichtung nach Anspruch 8, dadurch gekennzeichnet, daß die hohlen Fasern einen inneren Durchmesser zwischen 180 und 260 μm aufweisen, diese Werte eingeschlossen.

**15.** Vorrichtung nach Anspruch 8, dadurch gekennzeichnet, daß die hohlen Fasern eine Dicke zwischen ungefähr 50 und ungefähr 250 μm aufweisen.

**16.** Vorrichtung nach Anspruch 15, dadurch gekennzeichnet, daß die hohlen Fasern eine Dicke größer als 50 μm aufweisen.

**17.** Vorrichtung nach Anspruch 1, wenn das durch Adsorption wirkende Separatorelement aus einem Bündel hohler Fasern besteht, gekennzeichnet durch Fasern:

a) die einen inneren Durchmesser zwischen ungefähr 180 und ungefähr 260 μm aufweisen;
b) die aus einem homogenen und symmetrischen polyelektrolytischen Hydrogel bestehen;
c) die eine Permeabilität für Wasser Lp größer als ungefähr 5 ml/h/mmHg/m$^2$ haben;
d) die einen Membranwiderstand gegenüber Harnstoff kleiner als ungefähr 200 mih/cm haben und
e) die die Kapazität haben, mindestens eine der unter TNF-$\alpha$, Faktor D des Komplements oder Interleukin 1-$\beta$ gewählten Substanzen zu adsorbieren, wobei ihre Adsorptionskapazität pro Längeneinheit je nach dem Fall größer als ungefähr 125 pg/m für TNF-$\alpha$, größer als ungefähr 6 μg/m für Faktor D und größer als ungefähr 60 pg/m für Interleukin 1-$\beta$ ist.

**18.** Vorrichtung nach Anspruch 17, dadurch gekennzeichnet, daß das Hydrogel sich von einem Copolymer von Acrylnitril und mindestens einem ionischen oder ionisierbaren Monomer ableitet, das gegebenenfalls konstitutionelle Einheiten enthält, die von mindestens einem weiteren olefinisch ungesättigten Monomer stammen, das mit Acrylnitril copolymerisiert werden kann.

**19.** Vorrichtung nach Anspruch 17 oder 18, dadurch gekennzeichnet, daß das Hydrogel sich von einem Copolymer von Acrylnitril und Natrium-methallylsulfonat ableitet.

**20.** Vorrichtung nach Anspruch 19, dadurch gekennzeichnet, daß der Ionengehalt des Copolymers größer als oder gleich 400 mEq/kg, vorzugsweise größer als oder gleich 500 mEq/kg ist.

**21.** Vorrichtung nach Anspruch 19 oder 20, dadurch gekennzeichnet, daß das Copolymer ungefähr 3,3 Mol-% Natrium-. methallylsulfonat enthält und von der Firma HOSPAL unter der Marke AN 69 vertrieben wird.

Fig. 1

Temps en min

Fig. 2

Fig. 3